# EUROPEAN PATENT APPLICATION

(11) **EP 2 589 356 A1**
(43) Date of publication of application: **08.05.2013**
(21) Application number: 11800833.3
(22) Date of filing: 28.06.2011
(51) Int. Cl.: A61F 13/15, A61F 13/472

(54) **WRAPPING CONFIGURATION**

(30) Priority: 29.06.2010 JP 2010148362
(71) Applicant: Unicharm Corporation, Ehime 799-0111 (JP)
(72) Inventor: KINOSHITA, Hideyuki, Kanonji-shi Kagawa 769-1602 (JP); HARADA, Hiroyuki, Kanonji-shi Kagawa 769-1602 (JP); KUDO, Jun, Kanonji-shi Kagawa 769-1602 (JP); MINAMI, Mari, Kanonji-shi Kagawa 769-1602 (JP)
(74) Representative: Peter, Julian
(86) International application number: PCT/JP2011/064775
(87) International publication number: WO 2012/002369

(57) **Abstract**

In the disclosed wrapping configuration (100), wings (41) that extend from both sides in the width direction (W) are provided on an absorbent article (1). On first regions (51) on the clothing side of the wings (41) and second regions (50) on the clothing side of the backing sheet (12), adhesive for affixing the absorbent article (1) to underwear is applied. With the wings (41) folded toward the skin-contacting side of the surface sheet (11), a water-degradable release sheet (21a) is provided so as to cover the first regions (51). Wrapping sheet (2) is non-water-degradable, is peelable from the underwear-affixing adhesive and is provided so as to cover the second regions (50).

## Description

### [Technical Field]

The present invention relates to a package structure configured of an absorbent article and a package sheet for packaging the absorbent article individually.

### [Background Art]

Conventionally, there is known a package structure configured of an absorbent article and a package sheet for packaging the absorbent article individually (for example, see Patent Literatures 1 and 2).

For example, in the package structure described in Patent Literature 1, when the package sheet is opened, adhesives used for the absorbent article to attach to underwear are all exposed.

Further, in the package structure described in Patent Literature 2, when the package sheet is opened, adhesives used for the absorbent article arranged in wing portions and hip flap portions which configure the absorbent article to attach to underwear remain covered by a non-hydrolyzable release sheet and are not exposed.

### [Citation List]

### [Patent Literature]

PTL 1: Japanese Patent No. 3685935
PTL 2: Japanese Patent Application Publication No. 2009-136341

### [Summary of Invention]

However, the applicants faced the following problem as regards the above package structure.

In the package structure described in Patent Literature 1, the adhesives used for the absorbent article to attach to underwear are all exposed when the package sheet is opened, so that when the absorbent article is attached to underwear, the adhesives arranged in the hip flap portions are adhered to each other or the adhesives arranged in the wing portions are adhered to the absorbent article body due to a folding pattern given to the hip flap portions at the time of individual packaging, thereby leading to a problem that the absorbent article cannot be attached to underwear finely, which possibly causes the leakage or twist.

Further, in the package structure described in Patent Literature 2, the release sheet is divided from the package sheet, so that the adhesives used for the absorbent article to attach to underwear are not all exposed in a case where the package sheet is opened. However, this leads to a problem that multiple pieces of trash which cannot be flushed down the toilet are generated, which causes trouble in dealing with this trash.

Thus, the present invention has been achieved in view of the aforementioned problem, and an object thereof is to provide a package structure which is, when opening the package sheet, capable of preventing exposure of all the adhesives used for the absorbent article to attach to underwear while reducing generation of trash which cannot be flushed down the toilet.

A first feature of the present invention is summarized as a package structure comprising: an absorbent article; and a package sheet that packages the absorbent article individually, wherein: the absorbent article comprising: a liquid-permeable topsheet; a liquid-impermeable backsheet; and an absorber arranged between the topsheet and the backsheet; the absorbent article is provided with a wing portion protruded outwardly in a width direction; a first portion at a clothing contact surface side of the wing portion and a second portion at the clothing contact surface side of the backsheet are coated with an adhesive for fixing the absorbent article to underwear; a hydrolyzable release sheet is provided that covers the first portion in a state where the wing portion is folded back toward a skin contact surface side of the topsheet; and the package sheet is non-hydrolyzable, is releasable with respect to the adhesive for fixing to the underwear, and is provided that covers the second portion.

### [Brief Description of Drawings]

[Fig. 1] Fig. 1 shows a perspective view and a plan view of a package structure according to a first embodiment of the present invention.
[Fig. 2] Fig. 2 shows cross-sectional views taken along the line a-a and the line b-b of the package structure according to the first embodiment of the present invention.
[Fig. 3] Fig. 3 is a plan view of an absorbent article according to the first embodiment of the present invention, as seen from a skin contact surface side.
[Fig. 4] Fig. 4 is a plan view of the absorbent article according to the first embodiment of the present invention, as seen from a clothing contact surface side.
[Fig. 5] Fig. 5 is a plan view of an opened state of the package structure according to the first embodiment of the present invention, as seen from the skin contact surface side.
[Fig. 6] Fig. 6 is a plan view of the opened state of the package structure according to the first embodiment of the present invention, as seen from the clothing contact surface side.
[Fig. 7] Fig. 7 is a cross-sectional view taken along the line c-c of the opened state of the package structure according to the first embodiment of the present invention.
[Fig. 8] Fig. 8 is a view showing a state in which the absorbent article is taken out of the package structure according to the first embodiment of the present invention.
[Fig. 9] Fig. 9 is a plan view of an opened state of a package structure according to a first modification of the present invention, as seen from a skin contact surface side.
[Fig. 10] Fig. 10 is a plan view of an opened state of a package structure according to a second modification of the present invention, as seen from a skin contact surface side.
[Fig. 11] Fig. 11 is a cross-sectional view taken along the line a-a of the package structure according to the second modification of the present invention.

### [Description of Embodiments]

### (First embodiment of the present invention)

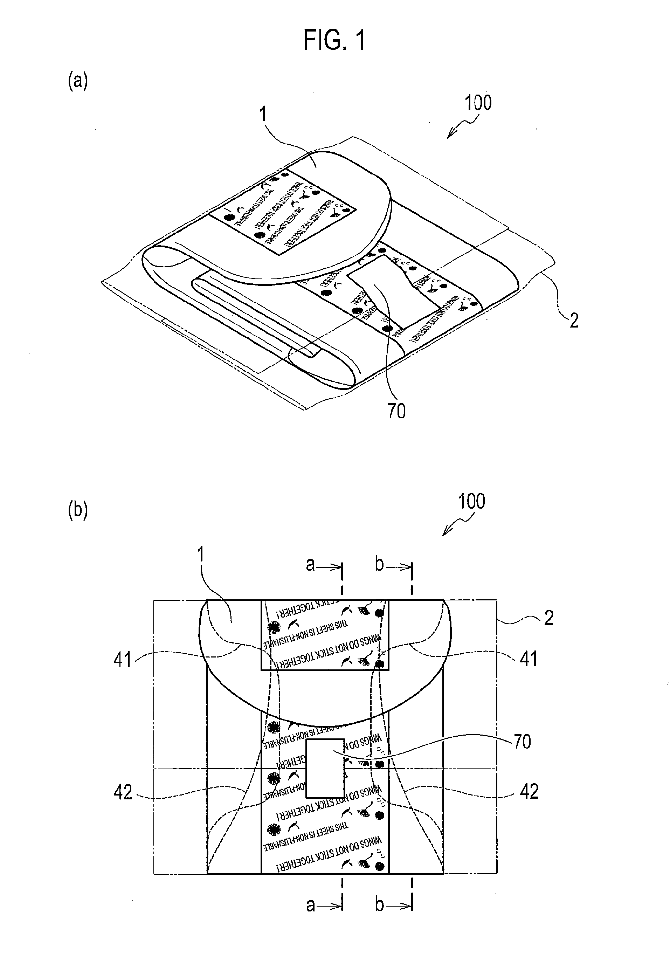

With reference to Figs. 1 to 8, a package structure 100 according to a first embodiment of the present invention will be explained. For example, an absorbent article 1 according to the present embodiment is a panty liner, an incontinence pad, sanitary napkin, or the like.

Fig. 1(a) shows a perspective view of the package structure 100 according to the present embodiment and Fig. 1(b) shows a plan view of the package structure 100 according to the present embodiment.

As shown in Figs. 1(a) and 1(b), the package structure 100 according to the present embodiment is configured of the absorbent article 1 and a non-hydrolyzable package sheet 2 for packaging the absorbent article 1 individually.

In the package structure 100 according to the present embodiment, the package sheet 2 is fixed with tape 70 while packaging the absorbent article 1 individually.

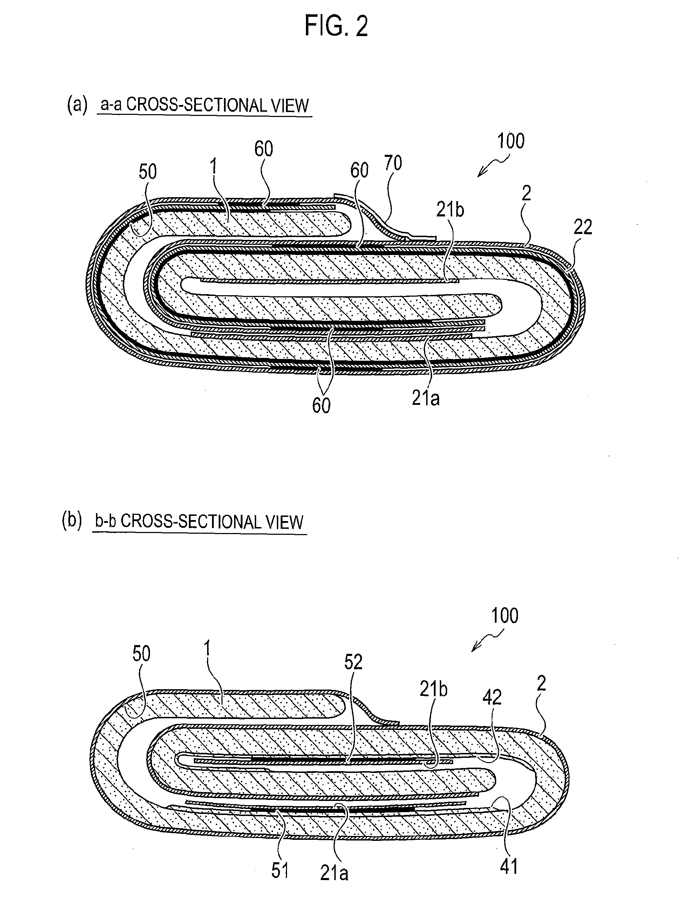

Figs 2(a) and 2(b) show cross-sectional views taken along the line a-a and the line b-b of the package structure 100 according to the present embodiment.

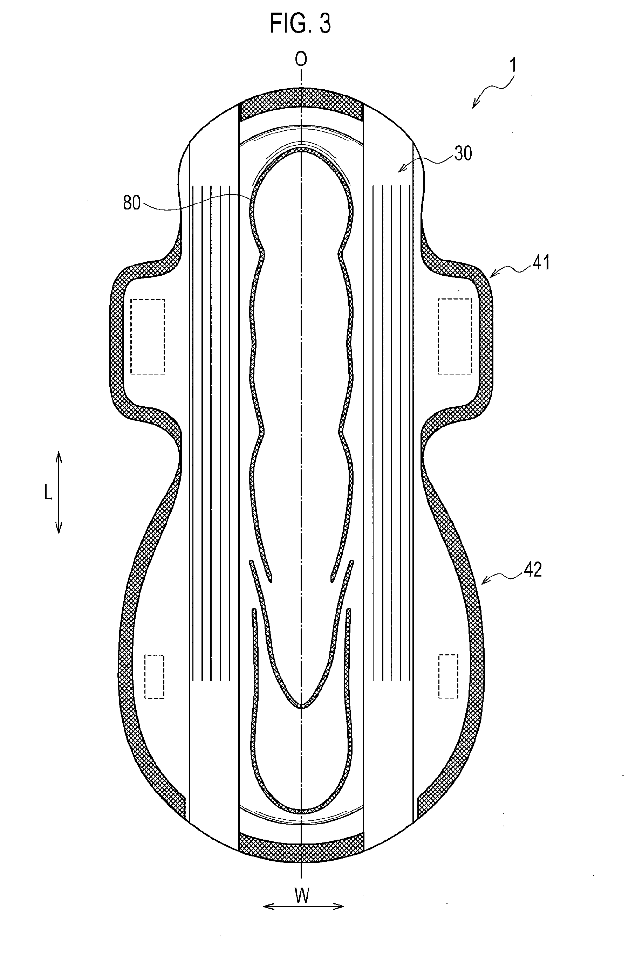

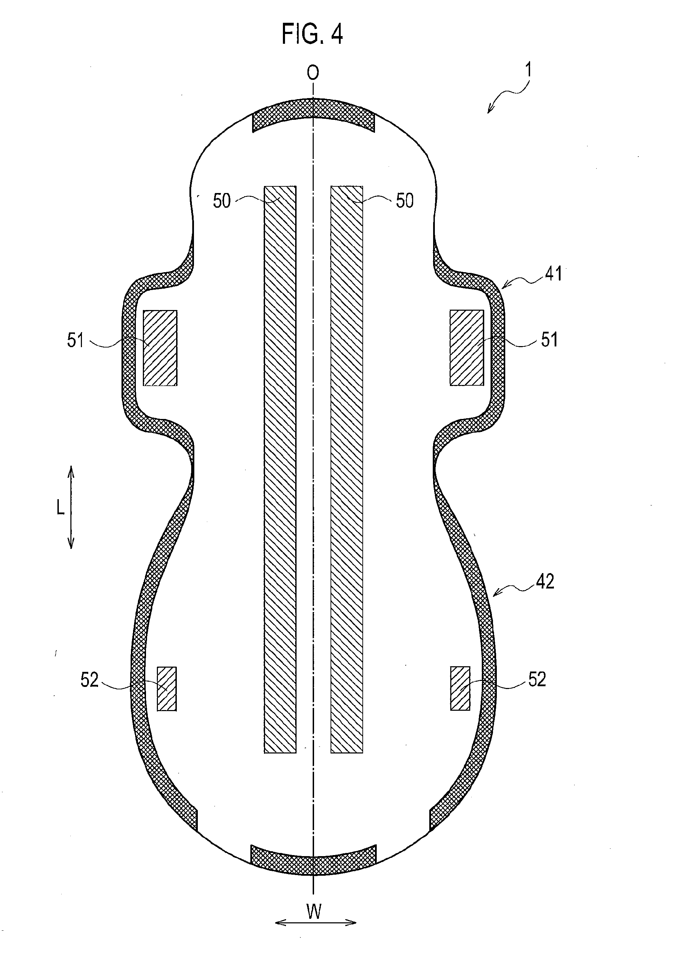

Fig. 3 shows a plan view of the absorbent article 1 configuring the package structure according to the present embodiment, as seen from a skin contact surface side, and Fig. 4 shows a plan view of the absorbent article 1 configuring the package structure according to the present embodiment, as seen from a clothing contact surface side.

As shown in Fig. 3, the absorbent article 1 is provided with wing portions 41 protruded outwardly in a width direction W. Furthermore, the absorbent article 1 according to the present embodiment has a pair of hip flap portions 42 in the rear of the wing portions 41 in a longitudinal direction L.

Herein, when attaching the absorbent article 1 to underwear, a user fixes the absorbent article 1 to the underwear by wrapping the wing portions 41 around a crotch of the underwear and spreading the hip flap portions 42 on the underwear.

Further, as shown in Fig. 3, the absorbent article 1 according to the present embodiment may be provided with a compressed groove 80 configured to have compression from a topsheet 10 to an absorber 13 in the longitudinal direction L.

Further, as shown in Fig. 3, the absorbent article 1 according to the present invention may have a barrier cuffs 30 configured of a barrier sheet 31 and an elastic member 32 at the outside in the width direction W of the absorber 13.

Further, as shown in Fig. 4, first portions 51 at the clothing contact surface side of the wing portions 41 are coated with adhesives for fixing the absorbent article 1 to underwear.

Further, second portions 50 at the clothing contact surface side of a backsheet 12 are coated with adhesives for fixing the absorbent article 1 to underwear.

Further, portions 52 at the clothing contact surface side of the hip flap portions 42 are coated with adhesives for fixing the absorbent article 1 to underwear.

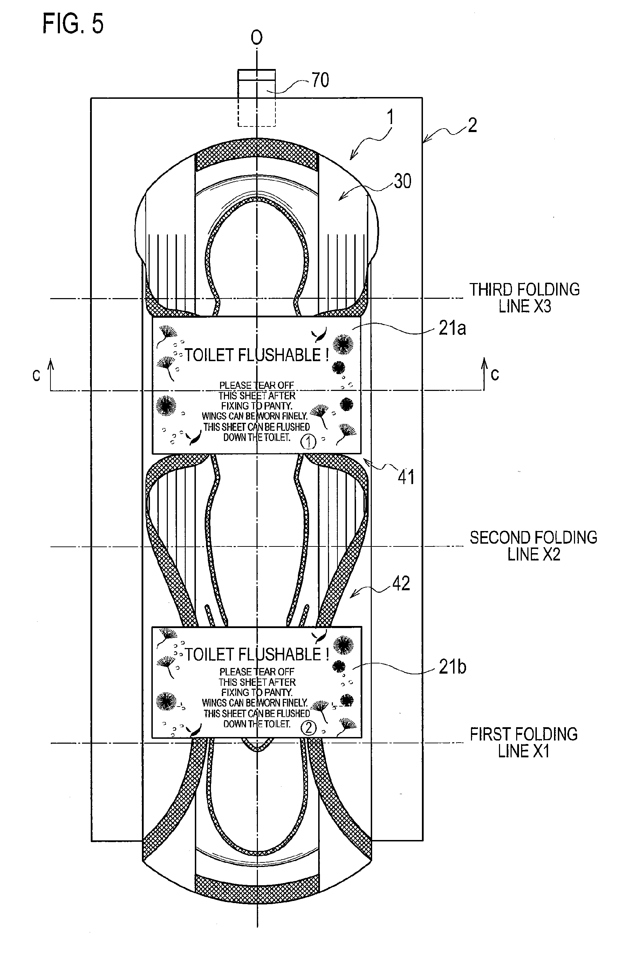

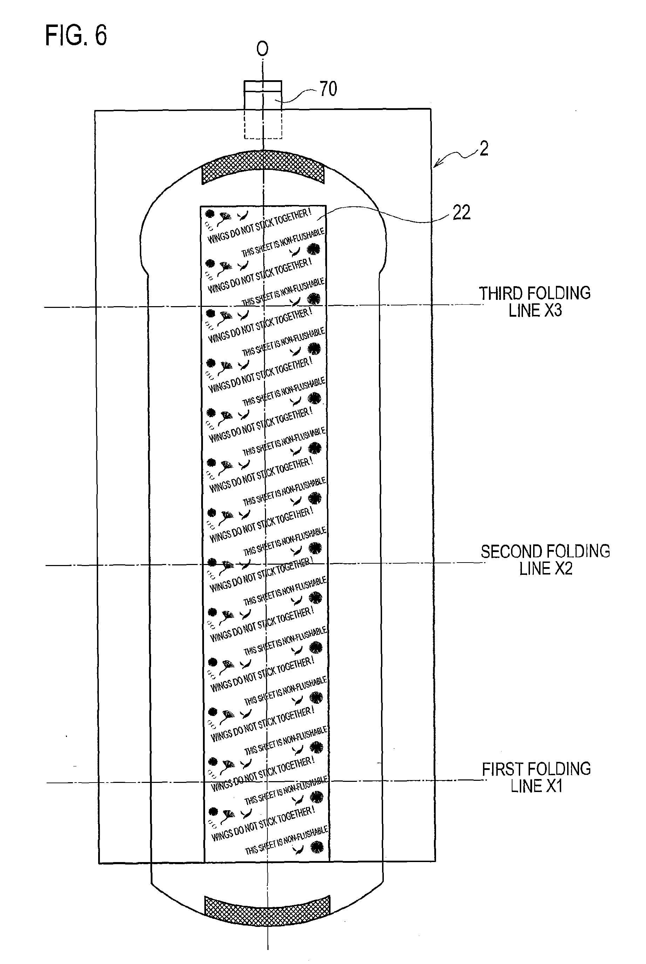

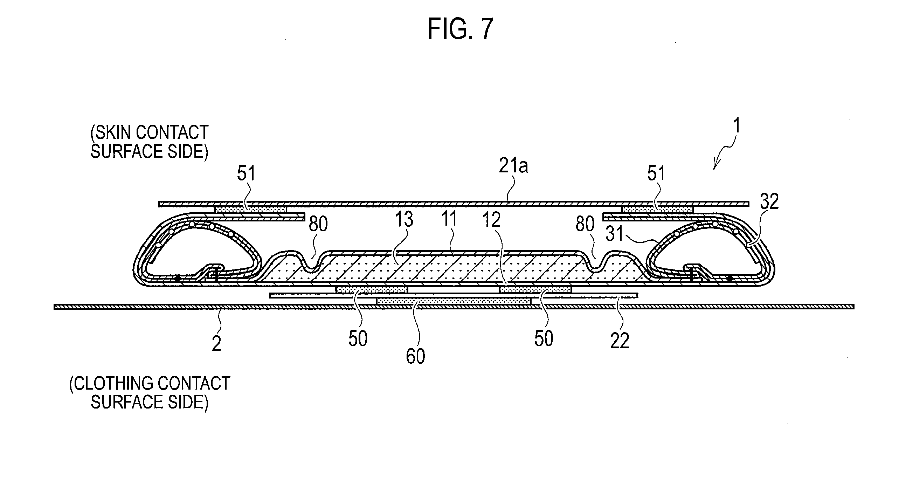

Fig. 5 shows a plan view of an opened state of the package structure according to the present embodiment, as seen from the skin contact surface side; Fig. 6 shows a plan view of the opened state of the package structure according to the present embodiment, as seen from the clothing contact surface side; and Fig. 7 shows a cross-sectional view taken along the line c-c of the opened state of the package structure according to the present embodiment.

As shown in Fig. 5, a hydrolyzable release sheet 21a is provided so as to cover the first portions 51 in a state where the wing portions 41 are folded back toward the skin contact surface side of the topsheet 11.

Similarly, a hydrolyzable release sheet 21b is provided so as to cover the portions 52 in a state where the hip flap portions 42 are folded back toward the skin contact surface side of the topsheet 11.

The release sheets for covering the first portions 51 and portions 52 are provided not integrally but independently, which requires less material to thereby achieve reduction in costs as well as improvement in hydrolyzability of the release sheets.

Herein, a material which can be used as the release sheets 21a, 21b is such as obtained by coating a surface of base paper of 36 g/m² (for example, 0.8 to 1.1 g/m³) made of pulp fibers with polyvinyle alcohol (PVA) of 1.0 to 2.0 g/m³ and further coating it over with silicone of 0.3 to 0.8 g/m³.

It is preferable that the hydrolyzable release sheets 21a, 21b be dispersed into three or more pieces within 24 hours by a shake-flask test to be described later.

Specifically, such a shake-flask test is carried out as described below.

In this shake-flask test, the release sheets 21a, 21b of 100 mm × 100 mm are used as a sample. Further, used in this shake-flask test are a shaking apparatus (such as a separatory funnel shaker manufactured by Asahi Techno Glass Corporation, Iwaki Glass Co., Ltd.), an eggplant shaped flask for 1000 mL, a measuring cylinder of 1000 mL, and the like.

Firstly, distilled water of 800 mL is poured into the eggplant shaped flask by using the measuring cylinder.

Secondly, the sample is put into the eggplant shaped flask.

Thirdly, the eggplant shaped flask is attached to the shaking apparatus. Herein, a screw-on lid side of the eggplant shaped flask is facing the bottom and a cock side of the eggplant shaped flask is facing the top.

Fourthly, a shaking condition (shaking speed: 240 rpm, shaking time: 24 hours) is set.

It is to be noted that in the present embodiment, the hydrolyzable release sheets 21a, 21b include water-soluble release sheets 21a, 21b.

For example, as the aforementioned water-soluble release sheets 21a, 21b, a material such as a film configured by water-soluble resin such as PVA, a material obtained by coating a nonwoven cloth with silicone, and the like can be used.

Further, as shown in Figs. 6 and 7, the package sheet 2 is releasable and also provided so as to cover the second portions 50.

Herein, a polyethylene sheet, a polypropylene sheet, a polyurethane sheet, and the like can be used as the package sheet 2, and polypropylene long-fibered nonwoven cloth laminated body with a spun bonding/melt-blowing/spun bonding structure can be used.

In the package structure 100 according to the present embodiment, as shown in Fig. 7, a non-hydrolyzable release sheet 22 as an independent body may be bonded to the package sheet 2 with an adhesive coated to a portion 60 at a side in which the absorbent article 1 is arranged.

In such a case, the release sheet 22 is provided so as to cover the second portions 50 at the clothing contact surface side of the backsheet 12.

Further, in the package structure 100 according to the present embodiment, a portion subjected to a release coating process using a release agent such as silicon resin or fluororesin system may be formed in at least one part of the package sheet 2.

Herein, at least one of different texts and patterns is inserted in the hydrolyzable release sheets 21a, 21b and the non- hydrolyzable release sheet 22 by means of printing, embossing, or the like.

For example, as shown in Fig. 5, at least one of texts and patterns indicative of a toilet flushable status, is inserted in the hydrolyzable release sheets 21a, 21b by means of printing, embossing, or the like.

Such an insertion is visually recognizable from the skin contact surface side of the hydrolyzable release sheets 21a, 21b in a state where the package structure 100 is opened.

Further, as shown in Fig. 6, at least one of a text and a pattern indicative of a toilet non-flushable status may be inserted in the non-hydrolyzable release sheet 22 by means of printing, embossing, or the like.

Such an insertion may be visually recognizable from a release surface side of the release sheet 22 in a state where the package structure 100 is opened and the package sheet 2 is released from the absorbent article 1. Further, such an insertion may be visually recognizable from the outside of the package structure 100 in a state where the absorbent article 1 is packaged individually.

Herein, at least one of a text and a pattern indicative of a toilet non-flushable status may be inserted in the package sheet 2 by means of printing, embossing, or the like.

Such an insertion may be visually recognizable from a release surface side of the package sheet 2 in a state where the package structure 100 is opened and the package sheet 2 is released from the absorbent article 1. Further, such an insertion may be visually recognizable from the outside of the package structure 100 in a state where the absorbent article 1 is packaged individually.

Further, the package structure 100 according to the present invention is configured so as to package the absorbent article 1 individually by folding the package sheet 2 along a fist folding line X1, a second folding line X2, and a third folding line X3, as shown in Figs. 5 and 6.

Herein, as shown in Fig. 5, the hydrolyzable release sheet 21a may be arranged so as not to cross the first folding line X1, the second folding line X2, and the third folding line X3 in the longitudinal direction L.

According to such a configuration, there is an improvement in visibility of a text or pattern inserted in the hydrolyzable release sheet 21a at the time of opening the package structure 100.

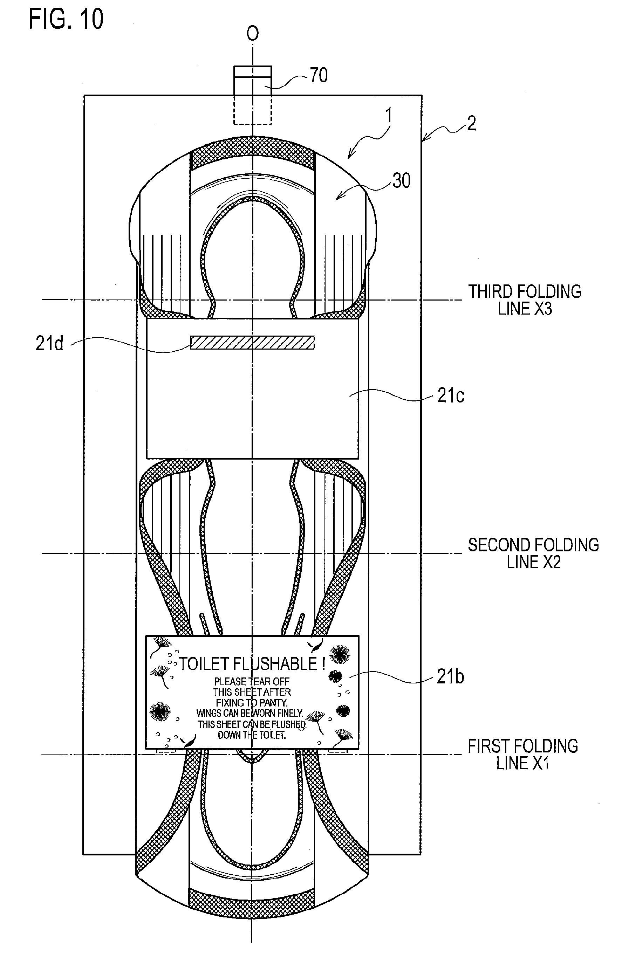

It is to be noted that the hydrolyzable release sheet 21b may also be arranged so as not to cross the first folding line X1, the second folding line X2, and the third folding line X3 in the longitudinal direction L (see Fig. 10 to be described in details later).

As shown in Fig. 7, the absorbent article 1 configuring the package structure 100 according to the present embodiment has the liquid-permeable topsheet 11, the liquid-impermeable backsheet 12, and the absorber 13 arranged between the topsheet 11 and the backsheet 12. It is to be noted that the absorbent article 1 according to the present embodiment may have a second sheet between the topsheet 11 and the absorber 13.

For example, as the topsheet 11, a fiber as an air-through nonwoven cloth with basis weight of 25, formed of a core with a thickness of 2.2 dtex made of polyethylene terephthalate (PET) and a clad made of polyethylene (PE) may be used.

Herein, a density of the topsheet 11 may be adjusted to be 0.025 g/cm³ by treating the topsheet 11 with a bulk recovery process (in which, for example, the topsheet 11 is left at a temperature of 90 degrees for 10 minutes).

The absorber 13 is configured by ground pulp, a superabsorbent polymer, and a core wrap for wrapping both. Herein, a macromolecular polymer is a granular polymer of a sodium acrylate copolymer.

Further, the core wrap is a sheet made from an arbitrary material which absorbs liquid. For example, as the core wrap, a tissue may be used, or an air-laid sheet formed by shaping a hydrophilic fiber or ground pulp into a sheet by an air-laid method may be used.

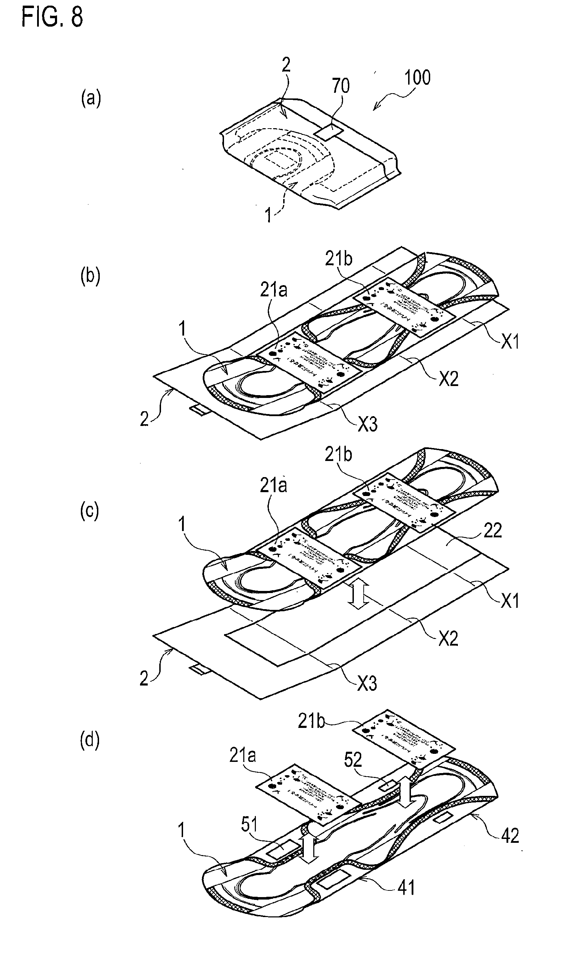

Described with reference to Figs. 8(a) to 8(d) is a movement of the user to use the absorbent article 1 included in the package structure 100 by opening the package structure 100 according to the present embodiment.

Firstly, the user removes a used absorbent article 1 fixed to underwear.

Secondly, as shown in Fig. 8(b), the user opens the package structure 100 in a state shown in Fig. 8(a).

Thirdly, as shown in Fig. 8(c), the user releases the package sheet 2 from the absorbent article 1.

Then the user wraps the used absorbent article 1 in the released package sheet 2 and carries it back or throws it into a trash box.

Further, in this absorbent article 1 in the state described above, the adhesives coated to the second portions 50 at the clothing contact surface side of the backsheet 12 are exposed whereas the adhesives coated to the first portions 51 at the clothing contact surface side of the wing portions 41 and the portions 52 at the clothing contact surface side of the hip flap portions 42 are not exposed.

Fourthly, the user attaches this absorbent article 1 to underwear.

Subsequently, as shown in Fig. 8(d), the user releases the hydrolyzable release sheet 21a from the absorbent article 1 and wraps the wing portions 41 around a crotch of the underwear to fix them. Herein, the user discards the released hydrolyzable release sheet 21a in a toilet.

Similarly, as shown in Fig. 8(d), the user releases the hydrolyzable release sheet 21b from the absorbent article 1 and spreads the hip flap portions 42 on underwear to fix them. Herein, the user discards the released hydrolyzable release sheet 21a in a toilet.

Next, some of methods of manufacturing the package structure 100 according to the present embodiment are described. It is to be noted that as far as the methods that are not described in below are concerned, the existing methods can be used.

Firstly, the absorbent article 1 is cut out of a web being conveyed.

Secondly, the wing portions 41 and the hip flap portions 42 are folded back toward the skin contact surface side of the absorbent article 1.

Thirdly, the release sheets 21a, 21b are coated with adhesives and arranged so as to cover the first portions 51 in the folded wing portions 41 and the portions 52 in the hip flap portions 42.

Fourthly, the absorbent article 1 is arranged on a web for the package sheet 2 in a state where the clothing contact surface side is facing the bottom.

Fifthly, a folding step is performed in a state where the absorbent article 1 is arranged on the web for the package sheet 2.

According to the package structure 100 in the present embodiment, the release sheets 21a, 21b are provided so as to cover the first portions 51 and the portions 52 in a state where the wing portions 41 are folded back toward the skin contact surface side of the topsheet 11 while the package sheet 2 and the release sheet 22 are provided so as to cover the second portions 50, so that the adhesives for attaching the absorbent article 1 to underwear can be prevented from being all exposed at the time of opening.

Further, according to the package structure 100 in the present invention, the non-hydrolyzable package sheet 2 and the release sheet 22 are bonded together, thereby being able to avoid such a situation that multiple pieces of trash which cannot be flushed down the toilet are generated at the time of opening.

Further, the package structure 100 according to the present embodiment enables the adhesives, which are arranged in the first portions 51 at the clothing contact surface side of the wing portions 41, to be exposed after the package sheet 2 is opened and then the adhesives arranged in the second portions 50 at the clothing contact surface side of the backsheet 12 are adhered to underwear, while enabling the release sheets 21a, 21b, which cover the adhesives arranged in the wing portions 41 and the hip flap portions 42, to be flushed down the toilet after the used absorbent article 1 is wrapped in the package sheet 2 and discarded.

Further, according to the package structure 100 in the present embodiment, at least one of different texts and patterns is inserted in the hydrolyzable release sheets 21a, 21b and the non-hydrolyzable release sheet 22. Specifically, in a case where at least one of texts and patterns indicative of a toilet flushable status is inserted in the hydrolyzable release sheets 21a, 21b while at least one of a text and a pattern indicative of a toilet non-flushable status is inserted in the non-hydrolyzable release sheet 22, the user can distinguish between the release sheets 21a, 21b which can be flushed down the toilet and the release sheet 22 which cannot be flushed down the toilet.

### (First modification)

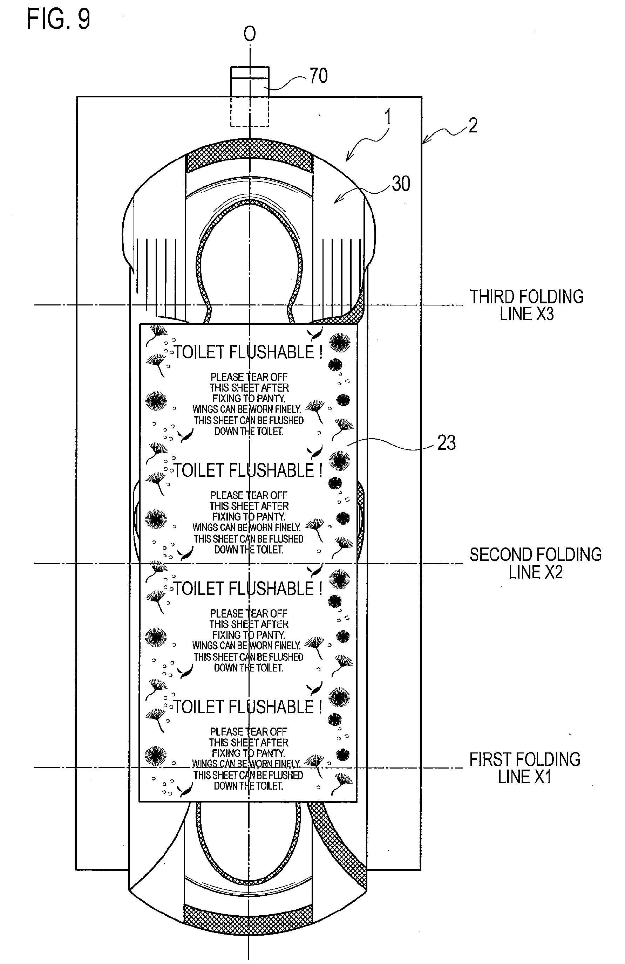

With reference to Fig. 9, the package structure 100 according to a first modification of the present invention will be described while focusing on the differences from the absorbent article 100 according to the aforementioned first embodiment.

In the package structure 100 according to this first modification, as shown in Fig. 9, in place of the hydrolyzable release sheets 21a, 21b, a pair of hydrolyzable release sheets 23 is provided so as to cover the portions 51 and the portions 52.

### (Second modification)

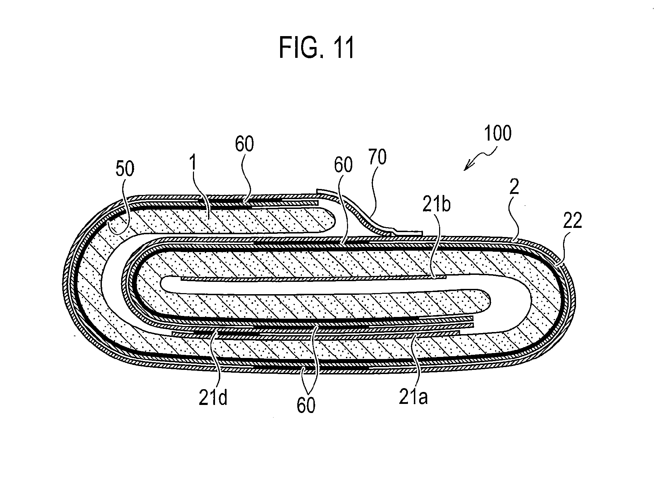

With reference to Figs. 10 and 11, the package structure 100 according to a second modification of the present invention will be described while focusing on the differences from the absorbent article 100 according to the aforementioned first embodiment.

In the package structure 100 according to this second modification, as shown in Figs. 10 and 11, an adhesive is coated to a portion 21d at a surface side not subjected to a release coating process, of a non-hydrolyzable release sheet 21c, in which the portion 21d comes in contact with the package sheet 2 in a state where the absorbent article 1 is packaged individually, and the release sheet 21c is provided so as to cover the portions 51

According to such a configuration, the non-hydrolyzable release sheet 21c is bonded to the package sheet 2 with the absorbent article 1 being packaged individually, thereby being released from the absorbent article 1 at the time of opening the package sheet 2 along the third folding line X3, which saves the user time and effort of releasing the non-hydrolyzable release sheet 21c from the absorbent article 1.

### (Other modifications)

It is to be noted that the hydrolyzable release sheets 21a, 21b and the non-hydrolyzable release sheet 22 may be configured of materials with different hues.

According to such a configuration, the user can distinguish between the release sheets 21a, 21b which can be flushed down the toilet and the release sheet 22 which cannot be flushed down the toilet.

It is to be noted that the hydrolyzable release sheets 21a, 21b and the non-hydrolyzable release sheet 22 may be given different hues by printing or by dyeing. In a case of giving different hues to the hydrolyzable release sheets 21a, 21b and the non-hydrolyzable release sheet 22 by dyeing, no ink is placed between fibers, thereby being able to maintain hydrolyzability of the release sheets 21a, 21b.

Further, the hydrolyzable release sheets 21a, 21b may be provided with a portion subjected to the release coating process and a portion not subjected to the release coating process.

For example, within the hydrolyzable release sheets 21a, 21b, a portion which comes in contact with the portions 51, 52 may be subjected to the release coating process and the other portion may not be subjected to the release coating process.

According to such a configuration, the portion not subjected to the release coating process is prone to get wet with water, thereby achieving improvement in hydrolyzability.

Further, the hydrolyzable sheets 21a, 21b may be formed by performing a release coating process to a crepe paper. According to such a configuration, its wave shape increases a surface area which comes in contact with water, thereby achieving improvement in hydrolyzability.

Further, the hydrolyzable sheets 21a, 21b may be provided with a plurality of slits or a stretch-processed region. According to such a configuration, it becomes easier for the hydrolyzable sheets 21a, 21b to be divided by water flow, thereby achieving improvement in hydrolyzability.

Further, the hydrolyzable sheets 21a, 21b may have a shape configured to reduce a resistance to water flow. For example, the hydrolyzable sheets 21a, 21b may have a shape having a convex portion in such as an arrowhead shape, as a shape to reduce a resistance to water flow.

According to such a configuration, it becomes easier for the hydrolyzable sheets 21a, 21b to sink under water, that is, to float away in water.

Further, the hydrolyzable sheets 21a, 21b may be coated with hydrophilic oil solution.

According to such a configuration, the hydrolyzable sheets 21a, 21b can be further improved in its hydrolyzability while making it easier for them to sink under water, that is, to float away in water.

It is to be noted that the absorbent article 1 included in the package structure 100 may not be provided with the wing portions 41. In such a case, the user fixes the absorbent article 1 to underwear by adjusting a position in which the absorbent article 1 has the shortest length in the width direction W, to a crotch of underwear at the time of wearing the absorbent article 1 to underwear.

Further, in consideration of attachability of the absorbent article 1 with respect to underwear, a length in the width direction W of the release sheets 21a, 21b, 22 may be shorter than a length in the width direction W of the absorber article 1.

Thus, the present invention has been explained in detail by using the above-described embodiments; however, it is obvious that for persons skilled in the art, the present invention is not limited to the embodiments explained herein. The present invention can be implemented as corrected and modified modes without departing from the gist and the scope of the present invention defined by the claims. Therefore, the description of the specification is intended for explaining the example only and does not impose any limited meaning to the present invention.

In addition, the entire content of Japanese Patent Application No. 2010-148362 (filed on June 29, 2010) is incorporated in the present specification by reference.

### [Industrial Applicability]

As described above, according to the present invention, it is possible to provide the package structure which is, when opening the package sheet, capable of preventing exposure of all the adhesives used for the absorbent article to attach to underwear while reducing generation of trash which cannot be flushed down the toilet.

### [Reference Signs List]

100... Package structure
1...Absorbent article
11...Topsheet
12... Backsheet
13... Absorber
21a, 21b, 22...Release sheet
30... Barrier cuffs
31... Barrier sheet
32... Elastic member
41... Wing portion
42... Hip flap portion

## Claims

1. A package structure comprising:
an absorbent article; and
a package sheet that packages the absorbent article individually, wherein:
the absorbent article comprising:
a liquid-permeable topsheet;
a liquid-impermeable backsheet; and
an absorber arranged between the topsheet and the backsheet;
the absorbent article is provided with a wing portion protruded outwardly in a width direction;
a first portion at a clothing contact surface side of the wing portion and a second portion at the clothing contact surface side of the backsheet are coated with an adhesive for fixing the absorbent article to underwear;
a hydrolyzable release sheet is provided that covers the first portion in a state where the wing portion is folded back toward a skin contact surface side of the topsheet; and
the package sheet is non-hydrolyzable, is releasable with respect to the adhesive for fixing to the underwear, and is provided that covers the second portion.

2. The package structure according to claim 1, wherein a non-hydrolyzable release sheet as a body independent of the package sheet is bonded to the package sheet.

3. The package structure according to claim 1, wherein a portion subjected to a release coating process is formed in at least one part of the package sheet.

4. The package structure according to claim 2, wherein at least one of different texts and patterns is inserted in the hydrolyzable release sheet and the non-hydrolyzable release sheet.

5. The package structure according to claim 4, wherein:
at least one of a text and a pattern indicative of a toilet flushable status is inserted in the hydrolyzable release sheet; and
at least one of a text and a pattern indicative of a toilet non-flushable status is inserted in the non-hydrolyzable release sheet.

6. The package structure according to any one of claims 2, 4 and 5, wherein the hydrolyzable release sheet and the non-hydrolyzable release sheet are configured of materials with different hues.

7. The package structure according to any one of claims 2 and 4 to 6, wherein the hydrolyzable release sheet is provided with a portion subjected to a release coating process and a portion not subjected to the release coating process.

8. The package structure according to any one of claims 2 and 4 to 7, wherein the hydrolyzable release sheet is formed by performing a release coating process to a crepe paper.

9. The package structure according to any one of claims 2 and 4 to 8, wherein the hydrolyzable release sheet is provided with a plurality of slits or a stretch-processed region.

10. The package structure according to any one of claims 2 and 4 to 9, wherein the hydrolyzable release sheet is coated with hydrophilic oil solution

11. The package structure according to any one of claims 2 and 4 to 10, wherein the hydrolyzable release sheet has an arrowhead shape.
